(19)

Europäisches
Patentamt
European
Patent Office
Office européen
des brevets

(11) **EP 2 783 634 A1**

(12) **EUROPEAN PATENT APPLICATION**
published in accordance with Art. 153(4) EPC

(43) Date of publication:
**01.10.2014 Bulletin 2014/40**

(21) Application number: **12851828.9**

(22) Date of filing: **25.05.2012**

(51) Int Cl.:
**A61B 8/06** (2006.01)

(86) International application number:
**PCT/JP2012/063484**

(87) International publication number:
**WO 2013/077013 (30.05.2013 Gazette 2013/22)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **25.11.2011 JP 2011258191**

(71) Applicant: **The University of Tokyo
Bunkyo-ku,
Tokyo 113-8654 (JP)**

(72) Inventors:
• **ONO Minoru**
  **Tokyo 113-8654 (JP)**
• **ITATANI Keiichi**
  **Tokyo 113-8654 (JP)**

(74) Representative: **Huebner, Stefan Rolf
SR HUEBNER & KOLLEGEN
Prinzregentenplatz 11
81675 Munich (DE)**

(54) **BLOOD FLOW VISUALIZING DIAGNOSTIC DEVICE**

(57)    A bloodstream visualizing diagnostic apparatus capable of visualizing and displaying a value relating to energy efficiency of bloodstream measured for an actual patient, and capable of improving usability, is provided. Provided is an bloodstream visualizing diagnostic apparatus which acquires flow rate information of a bloodstream flowing through a blood vessel in the body, calculates an energy loss of the bloodstream at a plurality of representative points within a region of interest in the blood vessel, using a calculation formula which does not explicitly include a blood pressure in the blood vessel, on the basis of the acquired flow rate information, and generates an image showing a magnitude of the calculated energy loss at the plurality of representative points.

```
                START

  S1 ┌─────────────────────────────┐
     │ Receive Bloodstream Flow Rate│
     │         Information           │
     └─────────────────────────────┘

  S2 ┌─────────────────────────────┐
     │      Calculate Energy Loss    │
     │   at Each Representative Point │
     └─────────────────────────────┘

  S3 ┌─────────────────────────────────────────┐
     │ Determine Pixel Value Corresponding to    │
     │ Each Representative Point on the Basis of  │
     │ Corresponding Energy Loss Calculation Result│
     └─────────────────────────────────────────┘

  S4 ┌─────────────────────────────┐
     │         Display Image         │
     └─────────────────────────────┘

                 END
```

Fig. 7

EP 2 783 634 A1

**Description**

Technical Field

The present disclosure relates to a bloodstream visualizing diagnostic apparatus capable of visualizing information relating to the bloodstream flowing through blood vessels in the body.

Background Art

Known technologies for visualizing the bloodstream flowing through blood vessels in the body may be a method using computer simulation, a method using an ultrasonic diagnostic apparatus, a method using an MRI (Magnetic Resonance Imaging), and the like.

Among these technologies, the method using computer simulation can be used for obtaining various information relating to a bloodstream, but in general, blood vessels of the patient to be actually measured are not used in this method, and thus, this method cannot be applied to diagnosis. According to the method using an ultrasonic diagnostic apparatus or an MRI, the actual state of a patient can be examined, and information regarding the flow rate of blood through blood vessels can be obtained. However, obtaining and visualizing the blood flow velocity is helpful for learning the state of the bloodstream, but is insufficient for obtaining information necessary for diagnosis.

For example, operation status of a prosthetic valve attached through surgery, etc., status of an aneurysmal portion located on a blood vessel, and so on, cannot be fully understood from the information regarding the blood flow velocity, and another viewpoint such as an energy efficiency of the bloodstream is necessary.

Patent Document 1 discloses a technology wherein computer simulation is performed using the shapes of blood vessels and bloodstream information actually measured by ultrasonic signals, and distributions of the bloodstream and blood pressures are calculated and displayed.

Prior Arts

Patent Document

Patent Document 1: Japanese Patent No. 4269623

Summary

As aforementioned, the prior arts have drawbacks that images necessary for making a diagnosis of an actual patient cannot be always obtained, and thus, are inferior in terms of usability.

One of the objects of the present disclosure is to provide a bloodstream visualizing diagnostic apparatus capable of visualizing and displaying values relating to the energy efficiency of the bloodstream measured in an actual patient, and capable of increasing usability.

The present disclosure is a bloodstream visualizing diagnostic apparatus comprising: an acquisition portion that is acquiring flow rate information of a bloodstream flowing through a blood vessel in the body; a calculation portion that is calculating an energy loss of the bloodstream at a plurality of representative points within a region of interest in the blood vessel, using a calculation formula which does not explicitly include a blood pressure in the blood vessel, on the basis of the acquired flow rate information; and an image generation portion that is generating an image showing a magnitude of the calculated energy loss at the plurality of representative points.

According to the present invention, usability in diagnosis can be increased.

Brief Description of Drawings

FIG. 1 is a block diagram showing a configuration example and a connection example of a bloodstream visualizing diagnostic apparatus according to an embodiment of the present disclosure.

FIG. 2 is an explanatory view showing an example of flow rate information of a bloodstream received by a bloodstream visualizing diagnostic apparatus according to an embodiment of the present disclosure.

FIG. 3 is a functional block diagram showing an example of a bloodstream visualizing diagnostic apparatus according to an embodiment of the present disclosure.

FIG. 4 is a schematic explanatory view explaining a content of calculation in a bloodstream visualizing diagnostic apparatus according to an embodiment of the present disclosure.

FIG. 5 is an explanatory view showing an example of a blood vessel to be diagnosed by a bloodstream visualizing diagnostic apparatus according to an embodiment of the present disclosure.

FIG. 6 is an explanatory view showing an example of a coordinate system which is used when a detection device connected to a bloodstream visualizing diagnostic apparatus according to an embodiment of the present disclosure, measures the flow rate.

FIG. 7 is a flowchart showing an example of operation of a bloodstream visualizing diagnostic apparatus according to an embodiment of the present disclosure.

Embodiment

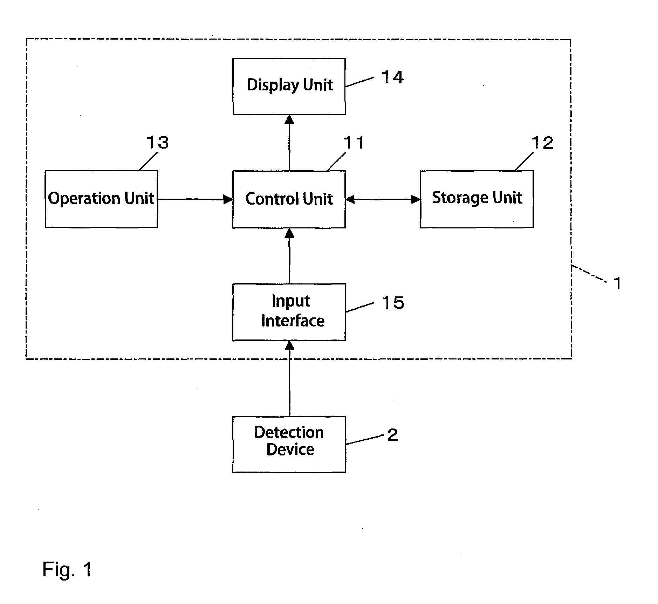

An embodiment of the present disclosure will be explained with reference to the drawings. As exemplified in FIG. 1, a bloodstream visualizing diagnostic apparatus 1 according to an embodiment of the present disclosure is connected to a detection device 2 which detects a flow rate of a bloodstream flowing through a blood vessel in the body. The bloodstream visualizing diagnostic apparatus 1 comprises a control unit 11, a storage unit 12, an operation unit 13, a display unit 14, and an input interface 15.

The control unit 11 is a program control device such as a CPU, and operates in accordance with a program stored in the storage unit 12. In the present embodiment, the control unit 11 acquires, from the detection device 2, flow rate information of a bloodstream flowing through a blood vessel in the body, and on the basis of the acquired flow rate information, calculates an energy loss of the bloodstream at a plurality of representative points within a region of interest in the blood vessel, using a calculation formula which does not explicitly include a blood pressure in the blood vessel. Then, the control unit 11 generates an image showing a magnitude of the calculated energy loss at the plurality of representative points, and outputs and displays the image. The specific operation of the control unit 11 will be explained in detail below.

The storage unit 12 comprises a memory device, a disk device, or the like. The storage unit 12 holds a program to be executed by the control unit 11. This program is provided while being stored in a computer readable and non-transitory recording medium such as a DVD-ROM (Digital Versatile Disc Read Only Memory), etc., and is copied into the storage unit 12. The storage unit 12 also functions as a work memory of the control unit 11.

The operation unit 13 is a mouse, a keyboard, and the like, which receives input of instructions from a user, and outputs information showing the content of the instructions to the control unit 11. The display unit 14 is a display device such as a liquid-crystal display. In accordance with the instructions input from the control unit 11, the display unit 14 outputs and displays the information. The input interface 15 accepts information relating to the flow rate of the bloodstream from the detection device 2, and outputs the information to the control unit 11.

The detection device 2 is, for example, an ultrasonic diagnostic apparatus which emits ultrasonic signals from the body surface side toward the blood vessel in the body, and receives the ultrasonic signals reflected in the body. Specifically, an ultrasonic diagnostic apparatus has a probe which scans within a predetermined range (region of interest) and emits ultrasonic signals at a frequency of f0 in respective directions. The ultrasonic signal emitted to a certain direction starts from the probe, is reflected by an erythrocyte within a blood vessel located in that direction, and returns to the probe. Since the Doppler shifting occurs corresponding to the moving speed of the erythrocyte, the frequency is shifted from f0 to f0+fd.

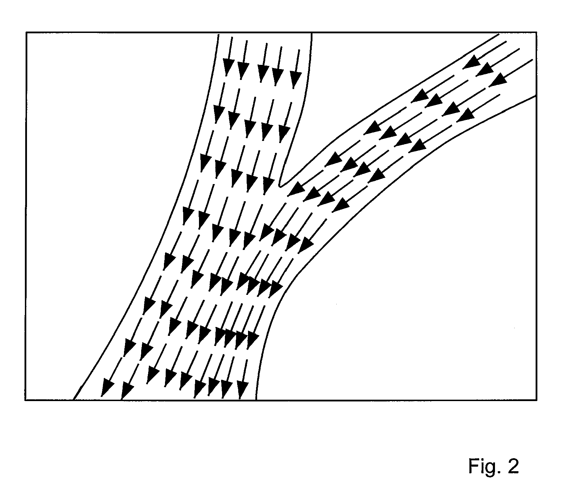

Using the fd (Doppler Shift Frequency), the moving speed of the erythrocyte, namely, the flow rate of the bloodstream can be detected. When ultrasonic signals are emitted while two-dimensional scanning is performed, flow rate information of a bloodstream located in respective directions on a two-dimensional surface can be obtained (FIG. 2). This method is known as, for example, Color Doppler Imaging (CDI), etc., and thus, detailed explanation thereon is omitted here.

Another example of the detection device 2 may be an MRI. An ordinary MRI visualizes a blood vessel, etc., by signal intensity (Magnitude Image), whereas an MRI phase image shows information in proportion to the blood flow velocity with magnetic field gradient direction, and enables the detection of direction and magnitude of a blood flow velocity at arbitrary point in space. By superimposing a series of phase images in respective directions of magnetic field gradient, and further superimposing the series of phase images onto the intensity image of a blood vessel shape, distribution of bloodstreams within a blood vessel can be visualized by vectors. This method is known as phase velocity mapping, and detailed explanation thereon is omitted here.

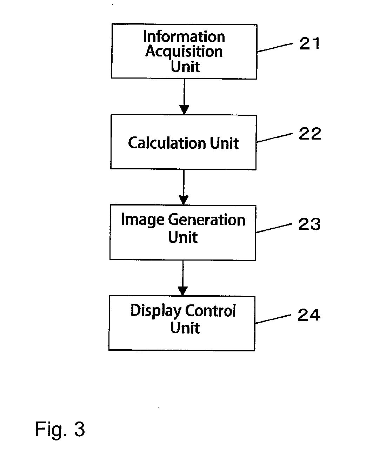

Next, the operation of the control unit 11 according to the present embodiment will be explained. As exemplified in FIG. 3, the control unit 11 according to the present embodiment functionally comprises an information acquisition unit 21, a calculation unit 22, an image generation unit 23, and a display control unit 24. The information acquisition unit 21 receives the flow rate information of the bloodstream output from the detection device 2. As exemplified above, this information is the flow rate information of bloodstreams located in respective directions on a two-dimensional surface (respective positions which can be represented by coordinate x1, x2). Here, the flow rate information can be represented by a vector (u1, u2, u3), each component of the vector being a component in the direction of each three-dimensional coordinate axis.

The calculation unit 22 refers to the received information of the bloodstream, and calculates an energy loss ΔE of the bloodstream at a plurality of representative points using the following Formula (1) which does not explicitly include a blood pressure in the blood vessel, on the basis of the flow rate information of the bloodstream at each representative point, the representative points being positions on a two-dimensional surface at which the flow rate information can be obtained.

[Formula 1]

$$\Delta E = \sum_{i,j} \int \frac{1}{2}\eta \left( \frac{\partial u_i}{\partial x_j} + \frac{\partial u_j}{\partial x_i} \right)^2 dV \qquad (1)$$

In Formula (1), $\eta$ represents a viscosity.

In Formula (1), integration over a predetermined volume range (V) is necessary. However, when calculating an energy loss ΔE of the bloodstream at each representative point, the integrand itself can be treated to express the energy loss ΔE, because Formula (1) can be integration over a local volume in which the result of the integrand is substantially constant. Namely, the calculation unit 22 calculates the integrand (differential can be calculated by replacing to difference) of Formula (1), and outputs the calculation result. Explanation on why this calculation formula represents the energy loss will be described below.

The image generation unit 23 determines a pixel value of a corresponding pixel on the two-dimensional surface, on the basis of the energy loss ΔE of the bloodstream at each representative point on the two-dimensional surface obtained by the calculation unit 22. For example, the pixel values are determined such that, the closer the energy loss ΔE to 0, the closer the color to blue, and as the energy loss ΔE increases, the color changes to green, yellow, orange, and red. The pixel value can be determined not on the basis of the energy loss ΔE, but on the basis of log ΔE, a logarithm of ΔE.

Thereby, the image generation unit 23 generates an image showing energy loss distribution, this image corresponding to a two-dimensional image generated by the ultrasonic diagnostic apparatus functioning as the detection device 2. The display control unit 24 outputs the image generated by the image generation unit 23 to the display unit 14 to display the image thereon.

Here, why the energy loss can be calculated by Formula (1) used by the calculation unit 22 is to be explained. The bloodstream is an incompressible viscous fluid. Thus, according to a known theory in fluid dynamics, Navier-Stokes equation can be satisfied.

[Formula 2]

$$\nabla \cdot u = 0 \qquad (2)$$

[Formula 3]

$$\rho \frac{\partial u}{\partial t} = -\rho \left( u \cdot \nabla \right) u - \nabla P + \eta \nabla^2 u \qquad (3)$$

In Formula (3), the external force is 0. P represents a hydrostatic pressure, $\rho$ represents a density, $\eta$ represents a viscosity, and t represents time. As mentioned above, the velocity u is a vector (u1, u2, u3) having components in three-dimensional coordinate axis directions. With respect to blood, the density $\rho$ is approximately 1060 kg/m$^3$, and the viscosity $\eta$ is from 3.0 to 5.0 kg/m/s.

Formula (3) (Navier-Stokes equation) does not explicitly include the energy of a fluid. The energy of a fluid decreases by dissipation due to viscosity of the fluid. This fluid energy can be expressed by Formula (4), in which e represents an internal energy.

[Formula 4]

$$\frac{1}{2}\rho|u|^2 + e = \frac{1}{2}\rho|u|^2 + \frac{TS}{V} + P \qquad (4)$$

Here, according to basic laws of thermodynamics, the internal energy can be expressed by Formula (4), wherein T represents a temperature, S represents entropy, V represents a volume, and P represents a pressure. Further, by differentiating Formula (4), Formula (5) can be obtained.
[Formula 5]

$$\mathrm{d}\left(\frac{1}{2}\rho|u|^2 + e\right) = \mathrm{d}\left(\frac{1}{2}\rho|u|^2\right) + \frac{T\mathrm{d}S - P\mathrm{d}V}{V} \qquad (5)$$

The bloodstream in the human body can be reasonably assumed as an adiabatic and incompressible flow, dS=0 and dV=0 are satisfied, and accordingly, Formula (5) can be simply expressed as Formula (6).
[Formula 6]

$$\mathrm{d}\left(\frac{1}{2}\rho|u|^2\right) \qquad (6)$$

Temporal change of the energy E in a volume can be expressed by Formula (7).
[Formula 7]

$$\frac{\partial}{\partial t}E = \frac{\partial}{\partial t}\left(\int \frac{1}{2}\rho|u|^2 \mathrm{d}V\right) \qquad (7)$$

Here, Formula (8) can be obtained from Formula (3).
[Formula 8]

$$\begin{aligned}\frac{\partial}{\partial t}\left(\frac{1}{2}\rho|u|^2\right) &= -\rho u(u\cdot\nabla)u - u\nabla P + u_i\frac{\partial\sigma_{i,j}}{\partial x_j} \\ &= -(u\cdot\nabla)\left(\frac{1}{2}\rho|u|^2 + P\right) + \nabla\cdot(u_i\cdot\sigma_{i,j}) - \sigma_{i,j}\frac{\partial u_i}{\partial x_j}\end{aligned} \qquad (8)$$

Further, Formula (9) can be obtained using Formula (2).
[Formula 9]

$$\frac{\partial}{\partial t}\left(\frac{1}{2}\rho|u|^2\right) = -\nabla\cdot\left(\frac{1}{2}\rho|u|^2 + P - u_i\cdot\sigma_{i,j}\right) - \sigma_{i,j}\frac{\partial u_i}{\partial x_j} \qquad (9)$$

In addition, Formula (10) is also satisfied.
[Formula 10]

$$\sigma_{i,j} = \eta \left( \frac{\partial u_i}{\partial x_j} + \frac{\partial u_j}{\partial x_i} \right) \tag{10}$$

When Formula (9) is integrated with respect to the total volume of the region of interest, such as blood vessels, Formula (11) can be obtained from Gauss's theorem and the equation of continuity of Formula (2).
[Calculation Formula 11]

$$\frac{\partial}{\partial t} \left( \int \frac{1}{2}\rho|u|^2 dV \right) = -\int \left( \rho|u|^2 + P \right) u \cdot n dA + \int \left( \sigma_{i,j} \cdot u \right) \cdot n dA$$
$$\sum_{i,j} \int \frac{1}{2}\eta \left( \frac{\partial u_i}{\partial x_j} + \frac{\partial u_j}{\partial x_i} \right)^2 dV \tag{11}$$

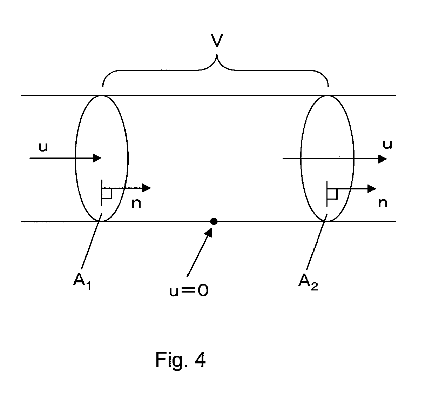

In Formula (11), n represents a normal vector standing on the surface of the region of interest, and A represents a surface area of the region of interest. Namely, the integration in the first term and the second term of the right-hand side is surface integral.
As shown in FIG. 4, it is reasonable to assume that the bloodstream has a flow rate of 0 at the blood vessel wall, and that a sufficient laminar flow can be found at the bloodstream inflow and outflow tracts (A1 and A2 in FIG. 4) of the region of interest (V), i.e., a linear portion having a constant diameter, for example, a peripheral blood vessel as a physiological blood vessel. In this case, the velocity u is directed perpendicular to the inflow surface, and it is reasonably assume that $\partial u_i/\partial n_i=0$ is satisfied on the surface.
Under these assumptions, in Formula (11), the second term of the right-hand side is 0. Therefore, Formula (11) can be simplified to Formula (12).
[Formula 12]

$$\frac{\partial}{\partial t} \left( \int \frac{1}{2}\rho|u|^2 dV \right) = -\int \left( \rho|u|^2 + P \right) u \cdot n dA + \sum_{i,j} \int \frac{1}{2}\eta \left( \frac{\partial u_i}{\partial x_j} + \frac{\partial u_j}{\partial x_i} \right)^2 dV \tag{12}$$

Further, the bloodstream receives influence from pulsation of the heart having a cycle T, and thus, the periodic boundary condition as in Formula (13) can be applied.
[Formula 13]

$$\int dt \left( \int \frac{1}{2}\rho|u|^2 dV \right) = \left( \int \frac{1}{2}\rho|u|^2 dV \right)_{t=T} - \left( \int \frac{1}{2}\rho|u|^2 dV \right)_{t=0} = 0 \tag{13}$$

Then, Formula (14) can be obtained from Formula (12) and Formula (13).
[Formula 14]

$$\int dt \int \left( \frac{1}{2}\rho|u|^2 + P \right) u \cdot n dA = \int dt \sum_{i,j} \int \frac{1}{2}\eta \left( \frac{\partial u_i}{\partial x_j} + \frac{\partial u_j}{\partial x_i} \right)^2 dV \tag{14}$$

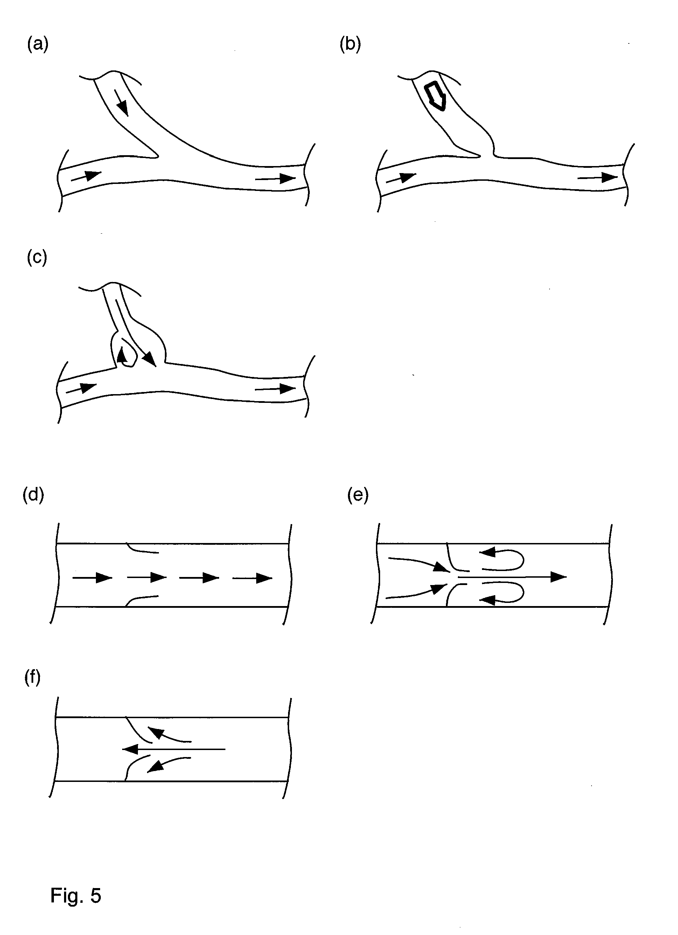

Under the reasonable assumptions relating to the bloodstream, Formula (14) indicates that the energy of fluid (left-hand side) is equal to the expression of the right-hand side. Accordingly, it has become understandable that the amount of energy change ΔE, i.e., the energy loss ΔE, of the bloodstream may be expressed by Formula (1).
Formula (14) may also make the following understandable. The left-hand side of Formula (14) may be also expressed by a sum of integrand values regarding the area A at the inflow tract and the outflow tract of the bloodstream. Namely, the gradient between the pressure P at the inflow tract and the pressure P at the outflow tract has an influence on the energy loss ΔE. Specific shapes of the blood vessels will be considered with reference to FIG. 5 (a) to FIG. 5 (g). FIG. 5 (a) shows an example of the shape of a healthy blood vessel. With respect to a blood vessel branched in Y-shape, when blood flows in from each branched blood vessel, FIG. 5 (a) shows smooth a bloodstream flow at the confluence. As a result, the pressure P on the inflow tract side is substantially equal to the pressure P on the outflow tract side. Accordingly, the energy loss is comparatively small.

However, as shown in FIG. 5 (b), if a stenosis occurs in the confluence zone from one blood vessel, the pressure P at the inflow tract side increases, while the pressure P at the outflow tract side decreases, resulting in a great energy loss. Also, as shown in FIG. 5 (c), if a blood vessel has an aneurysmal portion at the confluence zone, vortex of blood occurs at the aneurysmal portion. Thus, the pressure P at the inflow tract side decreases, resulting in a great energy loss. These are true when blood vessels are viewed locally, and thus, the energy loss is great at a position where a stenosis or an aneurysmal portion is found.

FIG. 5 (d) shows a healthy blood vessel in which valves can fully open. In this case, the value corresponding to the term for place-dependent velocity change shown on the right-hand side of Formula (14) becomes comparatively closer to 0 (the flow rate is almost unchanged at any places). Accordingly, the energy loss is comparatively small.

On the other hand, FIG. 5 (f) shows the state that the opening between the valves is small, and the blood vessel is narrow (the conditions that a stenosis occurs in the blood vessel). In this case, jet flow of the bloodstream from the narrow opening occurs. Accordingly, the velocity change becomes large at this place, resulting in having a comparatively large value of energy loss at the place where the valves exist.

Further, FIG. 5 (g) shows the state that valves cannot be closed, and regurgitant flow of blood occurs locally. In this case, convective flows are generated relative to the general flow of blood. Thus, velocity change emerges, resulting in having a comparatively large value of energy loss at the relevant portion.

As aforementioned, observing not the flow rate itself, but the energy loss ΔE, of the bloodstream, can easily specify the place where an abnormal bloodstream occurs due to an abnormal shape of the blood vessel, abnormal valves, etc. Namely, it can be understood that, at the place where the energy loss is large, an increased burden is put on the organ in order to get the bloodstream closer to normal. Thus, explanation to the patient becomes easy, and the doctor can easily specify the place having an abnormal bloodstream.

In the above explanation, the detection device 2 is an ultrasonic diagnostic apparatus, however, the present embodiment is not limited thereto. Namely, as far as the information regarding the flow rate of the bloodstream at respective parts within the region of interest is obtained, the energy loss of the respective parts can be obtained by calculating Formula (1). Specifically, the detection device 2 may be an MRI (Magnetic Resonance Imager).

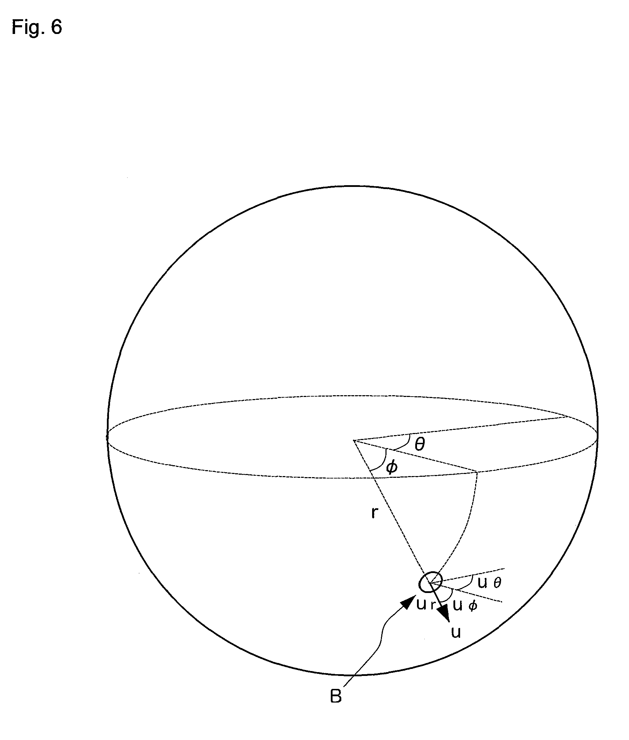

In addition, when the detection device 2 is an device to scan a region two-dimensionally extending from a probe, such as an ultrasonic diagnostic apparatus, the flow rate information of the bloodstream actually obtained by such a detection device 2 is expressed by polar coordinates with a radial direction defined by a line segment connecting the probe and an erythrocyte B to be observed (polar coordinates; r, θ, φ, with their origin at the position where the ultrasonic signal is received), as shown in FIG. 6.

Namely, the flow rate information of the bloodstream output from the detection device 2 and received by the information acquisition unit 21 is flow rate information of the bloodstream at respective directions on the two-dimensional surface (respective positions represented by coordinates x1, x2), but the flow rate information itself is a value of a component (u1, u2, u3) in Polar coordinate system regardless of the coordinates on the scanned surface.

Thus, the calculation unit 22 calculates Formula (1) in Polar coordinate system, Formula (1) being a formula which does not explicitly include the blood pressure in a blood vessel. Such coordinate transformation is known, and thus, detailed explanation thereon is omitted here. The calculation unit 22 calculates the energy loss ΔE (scalar quantity) of the bloodstream at a plurality of representative points, using Formula (1) in Polar coordinate system, on the basis of flow rate information of the bloodstream expressed in Polar coordinate system at each representative point, the representative point being a point at each position (position expressed in Cartesian coordinate system) on a two-dimensional surface at which the flow rate information can be obtained.

The image generation unit 23 determines a pixel value of a corresponding pixel on the two-dimensional surface, on the basis of the energy loss ΔE of the bloodstream at each representative point on the two-dimensional surface, obtained by the calculation unit 22. Thereby, the image generation unit 23 generates an image showing the magnitude of the energy loss at each of the plurality of representative points by transforming to Cartesian coordinate system. The display control unit 24 outputs the image generated by the image generation unit 23 to the display unit 14 to display thereon.

Further, when the detection device 2 is another device, the device calculates the energy loss ΔE using Formula (1) transformed to the same coordinate system as the velocity vector expressing the flow rate of the bloodstream detected at each representative point.

When the flow rate information of the bloodstream is obtained by phase velocity mapping of an MRI, Cartesian coordinate system may be used, depending on the direction of the phase image. In this case, the energy loss ΔE is obtained using Formula (1) in Cartesian coordinate system.

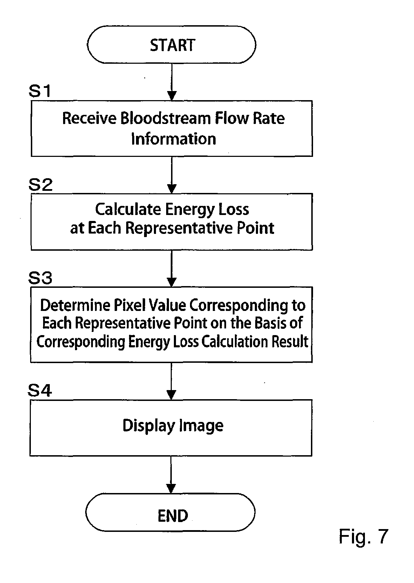

The present embodiment comprises the above-mentioned configuration, and the operation thereof is as follows. As shown in FIG. 7, the bloodstream visualizing diagnostic apparatus 1 receives flow rate information of the bloodstream output from the detection device 2 (S1). This flow rate information is obtained by relating vector values of flow rates at respective points Qi (coordinates (Xi, Yi)), (i=1,2,... N) on a two-dimensional surface (X-Y coordinate system).

Then, the bloodstream visualizing diagnostic apparatus 1 refers to the received information of the bloodstream, and calculates an energy loss ΔE(Qi) of the bloodstream at each of a plurality of representative points Qi on the two-dimensional surface on which flow rate information can be obtained, using Formula (1) which does not explicitly include blood pressure in the blood vessel, on the basis of the flow rate information of the bloodstream at each representative point Qi (S2). Here, as mentioned above, the integrand of Formula (1) is calculated (differential can be calculated by replacing to difference), and the value of the calculation result is output.

The bloodstream visualizing diagnostic apparatus 1 determines a pixel value a corresponding pixel on the two-dimensional surface (pixel at Qi coordinates (Xi, Yi)), on the basis of the logarithm of the energy loss ΔE(Qi), obtained in Process S2, of the bloodstream at each representative point Qi on the two-dimensional surface obtained (S3). For example, pixel values are determined so that the smaller the log ΔE, the logarithm of the energy loss ΔE, the closer the color to blue, and the as the log ΔE increases, the pixel values changes to have green, yellow, orange, and red. Then, the bloodstream visualizing diagnostic apparatus 1 outputs and displays the generated image (S4).

According to the present embodiment, the magnitude of the energy loss of the bloodstream at respective parts in the blood vessel, the energy loss having a direct relationship with the abnormality of the bloodstream, is calculated and displayed. Therefore, an image necessary for diagnosis of an actual patient can be obtained, and improved usability can be obtained.

Explanation on Numerals

1 bloodstream visualizing diagnostic apparatus, 2 detection device, 11 control unit, 12 storage unit, 13 operation unit, 14 display unit, 15 input interface, 21 information acquisition unit, 22 calculation unit, 23 image generation unit, 24 display control unit

## Claims

1. A bloodstream visualizing diagnostic apparatus comprising:

   an acquisition portion that is acquiring flow rate information of a bloodstream flowing through a blood vessel in the body,
   a calculation portion that is calculating an energy loss of the bloodstream at a plurality of representative points within a region of interest in the blood vessel, using a calculation formula which does not explicitly include a blood pressure in the blood vessel, on the basis of the acquired flow rate information, and
   an image generation portion that is generating an image generation means showing the magnitude of the calculated energy loss at the plurality of representative points.

2. An bloodstream visualizing diagnostic apparatus according to claim 1, wherein
   the acquisition portion acquires bloodstream information from a detector which detects, from the body surface side, flow rate information of the bloodstream flowing through a blood vessel in the body,
   the calculation portion calculates an energy loss of the bloodstream at a plurality of representative points within a region of interest in the blood vessel, using the calculation formula written in a coordinate system determined in relation to said detector, and
   the image generation portion generates an image showing the magnitude of the calculated energy loss at the plurality of representative points by transforming to Cartesian coordinate system.

3. An bloodstream visualizing diagnostic apparatus according to claim 2, wherein
   said detector is an ultrasonic detector which emits an ultrasonic signal from the body surface side toward a blood vessel in the body, receives the ultrasonic signal reflected in the body, and detects flow rate information of the bloodstream by the received ultrasonic signal, and
   said coordinate system determined in relation to the detector is polar coordinates with their origin at the receiving point of the ultrasonic signal by the ultrasonic detector.

Fig. 1

Fig. 2

| Information Acquisition Unit | 21 |
| Calculation Unit | 22 |
| Image Generation Unit | 23 |
| Display Control Unit | 24 |

Fig. 3

Fig. 4

(a)

(b)

(c)

(d)

(e)

(f)

Fig. 5

Fig. 6

START

S1

Receive Bloodstream Flow Rate
Information

S2

Calculate Energy Loss
at Each Representative Point

S3

Determine Pixel Value Corresponding to
Each Representative Point on the Basis of
Corresponding Energy Loss Calculation Result

S4

Display Image

END

Fig. 7

## INTERNATIONAL SEARCH REPORT

| International application No. |
|---|
| PCT/JP2012/063484 |

A. CLASSIFICATION OF SUBJECT MATTER
*A61B8/06*(2006.01)i

According to International Patent Classification (IPC) or to both national classification and IPC

B. FIELDS SEARCHED

Minimum documentation searched (classification system followed by classification symbols)
A61B8/00, 5/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2012 |
| Kokai Jitsuyo Shinan Koho | 1971-2012 | Toroku Jitsuyo Shinan Koho | 1994-2012 |

Electronic data base consulted during the international search (name of data base and, where practicable, search terms used)
PubMed, WPI

C. DOCUMENTS CONSIDERED TO BE RELEVANT

| Category* | Citation of document, with indication, where appropriate, of the relevant passages | Relevant to claim No. |
|---|---|---|
| X<br>Y | JP 63-222749 A (Aloka Co., Ltd.),<br>16 September 1988 (16.09.1988),<br>page 4, lower right column, line 16 to page 5,<br>upper left column, line 16<br>(Family: none) | 1<br>2,3 |
| Y | JP 2-177942 A (Yokogawa Medical Systems, Ltd.),<br>11 July 1990 (11.07.1990),<br>page 3, upper right column, line 17 to page 4,<br>upper right column, line 1<br>(Family: none) | 2,3 |

| ☒ Further documents are listed in the continuation of Box C. | ☐ See patent family annex. |
|---|---|

| | | |
|---|---|---|
| * Special categories of cited documents: | "T" | later document published after the international filing date or priority date and not in conflict with the application but cited to understand the principle or theory underlying the invention |
| "A" document defining the general state of the art which is not considered to be of particular relevance | | |
| "E" earlier application or patent but published on or after the international filing date | "X" | document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" document referring to an oral disclosure, use, exhibition or other means | | |
| "P" document published prior to the international filing date but later than the priority date claimed | "&" | document member of the same patent family |

| Date of the actual completion of the international search | Date of mailing of the international search report |
|---|---|
| 14 August, 2012 (14.08.12) | 28 August, 2012 (28.08.12) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.

PCT/JP2012/063484

C (Continuation).   DOCUMENTS CONSIDERED TO BE RELEVANT

| Category* | Citation of document, with indication, where appropriate, of the relevant passages | Relevant to claim No. |
|---|---|---|
| A | Fumiyoshi YUZAWA et al., "Nodo Kohaiho o Mochiita Bisho Kekkan Nai no Nijigen Sokudo Bunpu no Keisoku", The Transactions of the Institute of Electronics, Information and Communication Engineers, 1990.02, vol.J73-D-II, no.2, pages 276 to 282 | 1-3 |
| A | JP 11-104133 A  (Toshiba Corp.), 20 April 1999 (20.04.1999), entire text; all drawings (Family: none) | 2,3 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 4269623 B